(19) Europäisches Patentamt
European Patent Office
Office européen des brevets

(11) Numéro de publication : **0 007 882**
**B1**

(12) **FASCICULE DE BREVET EUROPÉEN**

(45) Date de publication du fascicule du brevet : **15.12.82**

(21) Numéro de dépôt : **79420033.7**

(22) Date de dépôt : **20.07.79**

(51) Int. Cl.³ : **C 07 C 33/03**, C 07 C 27/02, C 07 C 67/10, C 07 C 29/80, C 07 C 67/58, C 07 C 67/54

(54) **Procédé de préparation d'alcools allyliques à partir d'halogénures d'allyle.**

(30) Priorité : **24.07.78 US 927330**

(43) Date de publication de la demande : **06.02.80 (Bulletin 80/03)**

(45) Mention de la délivrance du brevet : **15.12.82 Bulletin 82/50**

(84) Etats contractants désignés : **BE CH DE FR GB IT NL**

(56) Documents cités :
**DE B 1 217 364**
**FR A 971 242**
**FR A 1 290 402**
**FR A 1 424 075**
**FR A 2 228 763**
**US A 4 091 039**
**US A 4 152 530**
**CHEMICAL ABSTRACTS, vol. 88, 1978 no. 5, January 30, page 457, ref. no. 37269n Columbus, Ohio, USA.**

(73) Titulaire : **RHONE-POULENC SPECIALITES CHIMIQUES**
**"Les Miroirs" 18, Avenue d'Alsace**
**F-92400 Courbevoie (FR)**

(72) Inventeur : **Gradeff, Peter S.**
**Forest Lakes**
**Andover New Jersey 07821 (US)**

(74) Mandataire : **Rioufrays, Roger et al**
**RHONE-POULENC RECHERCHES Centre de Recherches de Saint-Fons Service Brevets B.P. 62**
**F-69190 Saint-Fons (FR)**

**EP 0 007 882 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

# Procédé de préparation d'alcools allyliques à partir d'halogénures d'allyle

La présente invention a pour objet un procédé de préparation d'alcools allyliques par condensation d'un halogénure d'allyle avec un carboxylate alcalin en présence d'une base azotée avec formation d'un carboxylate d'allyle puis saponification de ce dernier par une base alcaline pour libérer l'alcool allylique avec formation d'un carboxylate alcalin qui est recyclé à la phase de préparation du carboxylate d'allyle.

Les halogénures d'allyle parmi lesquels on trouve les halogénures d'allyle terpéniques sont obtenus à partir de diverses sources, et représentent des intermédiaires de valeur pour la préparation d'esters et d'alcools allyliques qui sont utilisés en parfumerie et comme arômes. Les halogénures d'allyle terpéniques tel que le chlorhydrate de myrcényle constituent une matière première pour l'obtention du géraniol et du linalol, alors que les alcools allyliques des séries de la menthe verte peuvent être obtenus à partir du chlorure de carvyle et ceux des séries de la menthe poivrée à partir du chloro-5 menthène-3.

Dans le brevet américain n° 3 031 442 on a décrit la réaction d'un halogénure d'allyle terpénique avec un sel d'un acide carboxylique en présence d'une base azotée ou de ses sels comme catalyseur. Cette réaction n'exige pas la présence d'un solvant sauf pour faciliter l'agitation et le contact de l'halogénure d'allyle avec le sel solide. Tout solvant inerte peut être utilisé tel que les naphtas, les composés aromatiques ou les hydrocarbures saturés chlorés, les éthers, les cétones, les acides gras inférieurs correspondant à l'ester recherché. Toutefois ces acides gras inférieurs ne sont pas réellement inertes, car de grandes quantités abaissent l'activité des catalyseurs. Le produit de la réaction est un ester terpénique qui peut être hydrolysé en l'alcool allylique terpénique correspondant. Il est indiqué dans ce brevet que tout sel d'acide carboxylique peut être utilisé, mais l'acétate de sodium est préféré.

Dans le brevet américain n° 3 280 177 on a décrit une variante de ce procédé selon laquelle la réaction est conduite en présence de sulfoxyde de diméthyle comme catalyseur et comme solvant.

Dans le brevet anglais n° 1 459 622 on a proposé un perfectionnement aux procédés décrits dans les brevets américains mentionnés ci-avant, selon lequel la réaction de l'halogénure allylique terpénique avec le sel d'acide carboxylique en présence d'un catalyseur azoté est conduite en présence d'un composé polaire aprotique ayant un moment dipolaire d'au moins 3,1 Debye, une constante diélectrique supérieure à 15 et un point de fusion d'au moins 60 °C et/ou en présence d'ions iodure. Il est indiqué que les composés polaires aprotiques ont seulement une faible activité catalytique quand ils sont utilisés seuls, mais qu'ils amplifient considérablement l'activité catalytique des bases azotées. Comme exemples de composés polaires aprotiques on a cité les N,N-dialkylamides, les sulfoxydes, les nitriles, les dérivés nitrés, les carbonates, les esters phosphoriques, les phosphonamides.

Dans la demande française de brevet d'invention n° 74/16029 on a cité d'autres composés polaires aprotiques tels que les cétones (par exemple acétone) et les amides.

Dans la demande japonaise n° 77-10207 on a décrit un procédé de préparation du méthyl-3 butène-2 ol-1 par réaction du chloro-1 méthyl-3 butène-2 avec un sel d'acide carboxylique en présence d'une base azotée ou d'une base phosphorée pour former l'ester correspondant, puis hydrolyse de l'ester ainsi obtenu pour libérer le méthyl-3 butène-2 ol-1. La réaction est conduite en deux étapes avec séparation de l'ester intermédiaire du sel de l'acide carboxylique non transformé et du chlorure métallique formé au cours de la condensation.

La solution de l'ester et de l'halogénure d'allyle non transformé est ajoutée à une solution aqueuse d'hydroxyde de sodium et on procède à la saponification de l'ester.

Tous ces procédés ont en commun le problème de la récupération du sel alcalin d'acide carboxylique après saponification. Le rejet de la solution de ce sel et l'utilisation d'un sel neuf pour une nouvelle opération sont possibles mais peu intéressants du point de vue économique. En plus du coût relativement élevé du sel lui-même, il existe des problèmes inhérents au rejet de la solution de sel. La récupération du sel par évaporation de ses solutions aqueuses est bien connue mais entraîne des dépenses importantes en installation et en travail de manipulation du sel solide ainsi récupéré. Ainsi dans le brevet français 971 242 on a décrit un procédé de préparation de l'alcool allylique par condensation de l'acétate de sodium avec le chlorure d'allyle en présence d'eau et d'acide acétique puis saponification de l'acétate d'allyle qui implique la séparation préalable de ce dernier par distillation de son azéotrope avec l'eau, éventuellement sur un pied d'azéotrope eau/alcool allylique, puis la distillation de l'alcool formé et la récupération du carboxylate alcalin par évaporation de l'eau à sec de préférence.

Le but que la présente invention se propose d'atteindre consiste précisément à résoudre le problème de la récupération du carboxylate alcalin formé lors de la saponification et de son recyclage économique de façon à disposer d'un procédé cyclique de préparation des alcools allyliques dont chaque étape peut être conduite dans le même appareil.

La présente invention a donc pour objet un procédé de préparation d'alcools allyliques à partir d'halogénures d'allyle passant par la formation intermédiaire d'un ester, ledit procédé rendant inutile la séparation de l'ester allylique et permettant de réaliser la régénération du sel d'acide carboxylique dans le même appareillage de façon à éviter des opérations de séchage coûteuses impliquant le recours à des installations spéciales et à des manipulations. Ce procédé a pour résultat que l'halogénure d'allyle est transformé en alcool allylique en un minimum d'étapes et que le seul réactif consommé en dehors de l'halogénure d'allyle est une base alcaline.

Plus spécifiquement la présente invention a pour objet un procédé de préparation d'alcools allyliques par condensation, en présence d'un solvant organique non miscible à l'eau, d'un halogénure d'allyle de formule générale

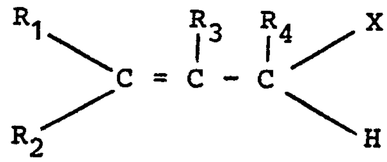

dans laquelle

a) $R_1$, $R_2$, $R_3$, $R_4$, identiques ou différents représentent de l'hydrogène, un radical alkyle ou alkényle,

b) l'un des radicaux $R_1$ ou $R_2$ a de 1 à 20 atomes de carbone, les autres radicaux $R_1$, $R_2$ et $R_3$, $R_4$ ayant de 1 à 5 atomes de carbone,

c) X est un atome de chlore, de brome ou d'iode, avec un carboxylate alcalin en présence d'une base azotée comme catalyseur en milieu anhydre et à une température comprise entre 50 et 150 °C, avec formation d'un carboxylate d'allyle, puis saponification de ce dernier à l'aide d'une base alcaline, avec formation d'alcool allylique et d'un carboxylate alcalin, caractérisé en ce que le solvant non miscible à l'eau est une cétone formant un azétorope avec elle, puis qu'on ajoute de l'eau à la masse réactionnelle pour dissoudre l'halogénure alcalin formé au cours de la condensation, sépare la solution aqueuse d'halogénure alcalin de la phase cétonique contenant le carboxylate d'allyle, ajoute ensuite une solution aqueuse d'un hydroxyde alcalin à la phase cétonique pour saponifier le carboxylate d'allyle par chauffage à une température comprise entre environ 50 °C et environ 150 °C, sépare les phases organiques et aqueuses du milieu de saponification, sépare l'alcool allylique par distillation de la phase organique et recycle éventuellement la cétone restante, ajoute de la cétone à la phase aqueuse de carboxylate alcalin résultant de la séparation et élimine l'eau par distillation azéotropique et enfin recycle la suspension cétonique de carboxylate alcalin ainsi obtenue à la phase de condensation.

L'alcool allylique est séparé de la cétone par distillation et peut être récupéré très pur et exempt de produit non transformé. Il n'y a pratiquement pas de réaction secondaire au cours des différentes étapes. De cette façon de très hauts rendements en alcool allylique peuvent être obtenus à partir de l'halogénure d'allyle.

Comme exemples de radicaux $R_1$, $R_2$, $R_3$ et $R_4$ on peut citer les radicaux méthyle, éthyle, n-propyle, isopropyle, n-butyle, isobutyle, pentyles, heptyles, décyles, méthyl-4 pentène-3 yle.

Comme exemple d'halogénures d'allyle, on peut citer le chloro-1 méthyl-3 butène-2 ; le chloro-1 diméthyl-3,7 octadiène-2,6 et le chloro-1 triméthyl-3,7,11 dodécyletriène-2,6,10.

Le sel alcalin d'acide carboxylique est de préférence un sel d'un acide carboxylique aliphatique ayant de 1 à 5 atomes de carbone ou d'un acide aromatique tels que les acides acétique, formique, propionique, butyrique, valérique, maléique, oxalique, tartrique, gluconique, succinique, malique, benzoïque et téréphtalique. Comme sel alcalin on peut utiliser les sels de sodium et de potassium. On fait appel de préférence aux acétates de sodium et de potassium.

Les bases azotées utilisées comme catalyseur sont connues et ne font pas partie de l'invention. On peut utiliser n'importe laquelle des bases azotées citées dans le brevet américain n° 3 031 442 telle que l'ammoniac, les amines, les amidines, les amides, les oximes, les hydroxylamides, les hydrazones, les semi-carbazides, les imines et leurs sels. Les amines peuvent être primaires, secondaires ou tertiaires ; il peut s'agir également de bases hétérocycliques. On utilise de préférence des bases azotées telles que les amines tertiaires : triméthylamine, triéthylamine, thiéthanolamine, tributylamine.

La cétone utilisée comme solvant à l'étape d'estérification et qui reste dans la couche organique durant la saponification peut être une cétone insoluble dans l'eau et formant un azéotrope avec elle. Les cétones inférieures solubles dans l'eau telles que l'acétone et la méthyléthylcétone ne forment pas d'azéotrope avec l'eau et ne peuvent convenir à cet égard. Parmi les cétones aliphatiques supérieures insolubles dans l'eau, on peut citer : la diisobutylcétone, la pentanone-3, la méthylhexylcétone, l'heptanone-2, la méthyl-2 heptène-2 one-6. Parmi les cétones aromatiques insolubles dans l'eau qui peuvent être utilisées, on peut citer : la benzophénone et l'acétophénone ; l'isophorone est un exemple de cétone cycloaliphatique qui convient à la mise en œuvre du procédé selon la présente invention. Si le carboxylate alcalin forme un hydrate, la cétone utilisée comme solvant doit avoir avoir un point d'ébullition supérieur à la température de décomposition de l'hydrate.

La concentration de l'ester ou de l'halogénure dans la cétone n'est pas critique. La quantité de cétone doit être suffisante pour donner un mélange réactionnel facile à agiter et il n'est pas nécessaire que la quantité utilisée à la phase de condensation soit la même que la quantité utilisée au cours de la distillation azéotropique de l'eau.

Dans certains cas, une quantité trop importante de cétone peut ralentir la vitesse d'hydrolyse. Par conséquent, pendant les étapes de condensation et d'hydrolyse, il est préférable que la quantité de cétone soit comprise entre 100 et 200 ml par molécule d'ester allylique ou d'halogénure d'allyle. Une partie de la cétone peut donc être éliminée s'il en reste plus que les quantités précitées après la

distillation azéotropique de l'eau.

Le procédé selon la présente invention peut être mis en œuvre dans un seul appareillage réactionnel duquel le carboxylate alcalin n'est jamais éliminé. L'appareil de réaction peut être utilisé successivement pour la condensation, la saponification et la distillation azéotropique de l'eau permettant le séchage du carboxylate alcalin formé au cours de la saponification. Après distillation azéotropique de l'eau le carboxylate alcalin est sec et prêt pour une nouvelle opération de condensation qui peut être conduite par addition d'une nouvelle charge d'alcool allylique et d'amine dans le réacteur.

La condensation est conduite à une température comprise entre environ 50° et environ 150 °C ; en général elle est terminée après une durée de réaction comprise entre quelques minutes et quelques heures. Par exemple entre quelques minutes et 10 heures. A une température comprise entre 90 et 130°, la réaction est complète entre 1 et 2 heures. La durée de la réaction est inversement proportionnelle à la température et les plus courtes durées de réaction sont obtenues aux températures les plus élevées.

Les quantités respectives de carboxylate alcalin et d'halogénure d'allyle sont voisines de la stœchiométrie, c'est-à-dire de 1 mole de carboxylate alcalin par mole d'halogénure d'allyle. Un léger excès de carboxylate alcalin, de l'ordre de 5 à 20 % en mole par rapport à la stœchiométrie est préféré pour conduire la réaction à son terme. Ainsi le rapport molaire halogénure d'allyle/carboxylate alcalin est généralement compris entre 1/1 et 1/1,2.

Le catalyseur est efficace en petite quantité. Ainsi on peut mettre en œuvre une quantité de base azotée représentant au moins 0,25 mole-% par rapport à l'halogénure d'allyle. On obtient les meilleurs résultats lorsque la quantité de catalyseur est comprise entre 1 et 10 % en mole par rapport à l'halogénure d'allyle mais on peut faire appel à des quantités plus importantes, par exemple jusqu'à environ 20 % si on le désire.

Lorsque la condensation est complète on ajoute une quantité d'eau suffisante pour dissoudre l'halogénure alcalin résultant de la réaction et la solution aqueuse d'halogénure alcalin est séparée et rejetée. La phase cétonique peut alors être soumise à la saponification.

La quantité d'hydroxyde alcalin utilisée au cours de la saponification est au moins égale à la quantité stœchiométrique mais un léger excès peut être également mis en œuvre. Le rapport molaire ester/base alcaline peut être compris entre 1/1 et 1/1,1.

Comme base alcaline on peut faire appel à tout hydroxyde alcalin tel que les hydroxydes de sodium et de potassium.

L'hydroxyde alcalin est utilisé sous forme de solution aqueuse. Bien que la concentration de ces solutions ne soit pas critique, on préfère avoir recours à des solutions contenant de 20 à 50 % en poids de base alcaline qui introduisent moins d'eau dans le milieu, de sorte que la quantité d'eau à éliminer pendant la phase de récupération du carboxylate alcalin est moindre.

L'hydrolyse est conduite à une température comprise entre 50 et 150 °C et de préférence entre 75 et 125 °C. On obtient de cette façon des rendements quantitatifs.

L'alcool allylique se forme très rapidement. En général la réaction est terminée après une durée comprise entre environ 0,5 h et environ 10 h. A une température comprise entre 110 et 120 °C, la durée de réaction est comprise entre 45 mn et 90 mn.

A la fin de la saponification, la phase aqueuse contient le carboxylate alcalin et l'hydroxyde alcalin dont la quantité dépend de l'excès mis en œuvre. La phase organique contient l'alcool allylique, la plus grande partie de la cétone et les produits non transformés tels que l'ester et l'halogénure allylique. Pour séparer ces différents composés, la phase cétonique est séparée de la phase aqueuse après décantation puis distillée. Si la cétone et la base azotée utilisées comme catalyseurs ont des points d'ébullition suffisamment différents de celui de l'alcool allylique, elles peuvent être séparées par distillation fractionnée.

Avant traitement de la phase aqueuse on procède à la neutralisation de l'hydroxyde alcalin en excès. Bien que tout acide carboxylique puisse être utilisé à cet effet, on préfère avoir recours à celui dont le sel alcalin est mis en œuvre à la phase d'estérification.

Après avoir ajouté une quantité suffisante de cétone pour distiller azéotropiquement l'eau, on procède à cette distillation jusqu'à ce que la totalité de l'eau ait été éliminée. Le sel anhydre est ensuite prêt pour un nouveau cycle de condensation.

Les exemples qui suivent illustrent l'invention et montrent comment elle peut être mise en pratique.

## Exemple 1

Dans un ballon à 3 cols de 1 litre, équipé d'un condenseur, d'un agitateur mécanique, d'un thermomètre et d'un piège à glace carbonique, on charge :

— 180,5 g d'acétate de sodium (2,2 moles)
— 161,6 g de diisobutylcétone
— 218,8 g de chloro-1 méthyl-3 butène-2 (2 moles).

On met l'agitation en marche puis on chauffe le mélange à reflux et ajoute 1 g de triéthylamine. Pendant que la réaction se déroule, on enregistre la disparition du chloro-1 méthyl-3 butène-2 par

chromatographie gaz-liquide.

Quand le taux de conversion atteint 95 %, on ajoute à nouveau 1 g de triéthylamide. Lorsque le taux de conversion du chloro-1 méthyl-3 butène-2 atteint 99,5 %, on ajoute 340 ml d'eau à la masse réactionnelle.

La phase aqueuse est séparée à température supérieure à 80 °C et rejetée. La couche cétonique est portée à 120 °C puis on lui ajoute 208,7 g d'une solution aqueuse de soude à 33,93 % en poids (2,05 moles), en 20 minutes, le mélange réactionnel étant maintenu à reflux pendant la durée de l'addition. On poursuit le chauffage à reflux pendant 1 heure, temps au bout duquel on constate, sur un échantillon, que la signification de l'acétate d'allyle intermédiaire en méthyl-3 butène-2 ol-1 est pratiquement terminée. La couche organique est séparée à 80-90 °C ; elle pèse 327,8 g.

La couche cétonique est introduite dans un ballon de distillation de 500 ml et distillée sous pression réduite, d'abord à 130 mm de mercure et à une température de 250 °C, puis on augmente progressivement la température et on diminue progressivement la pression de façon à obtenir une température des vapeurs de 77 °C et une température du pied de 108 °C sous une pression de 2,5 mm de mercure.

Dans le distillat, on dose par chromatographie gaz/liquide :

| | |
|---|---|
| — méthyl-3 butène-2 ol-1 : | 49 % en poids (156,2 g) |
| — méthyl-3 butènol-3 : | 1,1 % en poids ( 3,5 g) |
| — dichloro-2,4 méthyl-2 butène : | 0,9 % en poids ( 2,8 g) |
| — diisobutylcétone : | 39 % en poids (122,6 g). |

Le rendement basé sur le chloro-1 méthyl-3 butène-2 s'élève à 90,5 %.

La couche aqueuse (290 g) est ensuite chauffée à 90°, neutralisée par addition d'acide acétique anhydre jusqu'à pH 7,5 puis on ajoute de la diisobutylcétone. On procède ensuite à la distillation azéotropique de l'eau et de la cétone. L'eau est recueillie et la cétone renvoyée dans le pied de distillation. Quand la totalité de l'eau a distillé, on ajuste la quantité de cétone et un nouveau cycle de condensation est répété par addition de 218,8 g de chloro-1 méthyl-3 butène-2.

Le mélange est agité et porté à 115° puis on ajoute 1 g de triéthylamine. Lorsque la réaction est complète à 95 %, on ajoute à nouveau 1 g de triéthylamine puis lorsque le taux de transformation du chloro-1 méthyl-3 butène-2 atteint 99,5 % on introduit 340 ml d'eau et la masse réactionnelle ainsi obtenue est traitée comme précédemment.

La couche cétonique est hydrolysée comme ci-dessus et on récupère 329 g de couche organique contenant 48 % en poids de méthyl-3 butène-2 ol-1.

## Exemples 2 à 5

On opère comme à l'exemple 1 mais en remplaçant le carboxylate alcalin et l'hydroxyde alcalin ou la cétone par les composés indiqués dans le tableau ci-après. Les autres conditions sont également indiquées dans ce tableau et les tableaux suivants.

### A — CONDENSATION

| Exemples N° | Carboxylate alcalin* | Moles | Halogénure d'allyle | Moles | Amine g | Cétone | ml | Temp. °C | Durée heures | Halogénure non transformé % |
|---|---|---|---|---|---|---|---|---|---|---|
| 2 | formiate de sodium | 1,1 | chloro-1 méthyl-3 butène-2 | 1,0 | 3 | acétophénone | 100 | 90-95 | 4,0 | 2,7 |
| 3 | acétate de potassium | 1,1 | chloro-1 méthyl-3 butène-2 | 1,0 | 1 | acétophénone | 100 | 100 | 1,5 | 0,1 |
| 4 | acétate de sodium | 5,5 | chloro-1 méthyl-3 butène-2 | 5,0 | 5 | méthylhexyl-cétone | 928 | 80 | 11 | 0,2 |
| 5 | acétate de potassium | 1,2 | chlorure de géranyle + chlorure de néryle | 1,0 | 3 | acétophénone | 100 | 100 | 6 | 1,0 |

* Dans une série de condensations, le carboxylate utilisé est celui récupéré à l'étape C.

B — SAPONIFICATION

| Exemples N° | Base | Moles | Eau g | Température °C | Durée heures |
|---|---|---|---|---|---|
| 2 | NaOH | 1,1 | 102 | 100 | 2 |
| 3 | KOH | 1,1 | 62 | 100 | 2 |
| 4 | NaOH | 5,5 | 1 320 | 110 | 2 |
| 5 | KOH | 1,1 | 62 | 110 | 2 |

C — DISTILLATION AZEOTROPIQUE**

| Exemples N° | Cétone | ml | Température °C |
|---|---|---|---|
| 2 | acétophénone | 200 | 110-130 |
| 3 | acétophénone | 150 | 110-120 |
| 4 | méthylhexylcétone | 700 | 110-125 |
| 5 | acétophénone | 150 | 105-120 |

** Avant distillation l'excès de base est neutralisé par l'acide carboxylique correspondant.

Dans ces conditions, on a obtenu les résultats suivants :

| Exemples N° | Alcool allylique | Rendement % |
|---|---|---|
| 2 | Méthyl-3 butène-2 ol-1 | 89 |
| 3 | Méthyl-3 butène-2 ol-1 | 98 |
| 4 | Méthyl-3 butène-2 ol-1 | 95 |
| 5 | Géraniol et nérol | 90 |

**Revendications**

1. Procédé de préparation d'alcools allyliques par condensation, en présence d'un solvant organique non miscible à l'eau, d'un halogénure d'allyle de formule générale

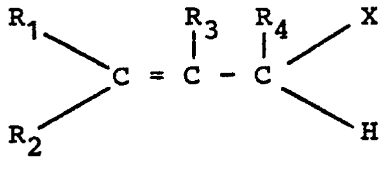

dans laquelle

a) $R_1$, $R_2$, $R_3$, $R_4$, identiques ou différents représentent de l'hydrogène, un radical alkyle ou alkényle,

b) l'un des radicaux $R_1$ ou $R_2$ a de 1 à 20 atomes de carbone, les autres radicaux $R_1$, $R_2$ et $R_3$, $R_4$ ayant de 1 à 5 atomes de carbone,

c) X est un atome de chlore, de brome ou d'iode avec un carboxylate alcalin en présence d'une base azotée comme catalyseur en milieu anhydre et à une température comprise entre 50 et 150 °C, avec formation d'un carboxylate d'allyle, puis saponification de ce dernier à l'aide d'une base alcaline, avec formation d'alcool allylique et d'un carboxylate alcalin, caractérisé en ce que le solvant non miscible à l'eau est une cétone formant un azétorope avec elle, puis qu'on ajoute de l'eau à la masse réactionnelle pour dissoudre l'halogénure alcalin formé au cours de la condensation, sépare la solution aqueuse d'halogénure alcalin de la phase cétonique contenant le carboxylate d'allyle, ajoute ensuite une solution aqueuse d'un hydroxyde alcalin à la phase cétonique pour saponifier le carboxylate d'allyle par chauffage à une température comprise entre environ 50 °C et énviron 150 °C, sépare les phases organiques et aqueuses du milieu de saponification, sépare l'alcool allylique par distillation de la phase organique et

recycle éventuellement la cétone restante, ajoute de la cétone à la phase aqueuse de carboxylate alcalin résultant de la séparation et élimine l'eau par distillation azéotropique et enfin recycle la suspension cétonique de carboxylate alcalin ainsi obtenue à la phase de condensation.

2. Procédé selon la revendication 1, caractérisé en ce que le carboxylate alcalin est un sel alcalin d'un acide aliphatique ayant de 1 à 5 atomes de carbone.

3. Procédé selon la revendication 2, caractérisé en ce que le carboxylate alcalin est un acétate de sodium et de potassium.

4. Procédé selon les revendications 1 à 4, caractérisé en ce que la cétone est choisie dans le groupe formé par la diisobutylcétone, la pentanone-3, la méthylhexylcétone, l'heptanone-2, la benzophénone, l'acétophénone.

## Claims

1. Process for the preparation of allyl alcohols by the condensation, in the presence of a water-immiscible organic solvent, of an allyl halide of the general formula

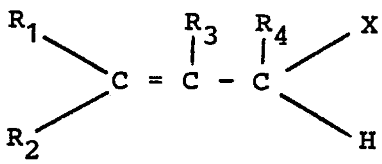

in which

a) $R_1$, $R_2$, $R_3$ and $R_4$, which are identical or different, represent hydrogen or an alkyl or alkenyl radical,

b) one of the radicals $R_1$ or $R_2$ has from 1 to 20 carbon atoms, the other radicals $R_1$ or $R_2$ and $R_3$ and $R_4$ having from 1 to 5 carbon atoms, and

c) X is a chlorine, bromine or iodine atom, with an alkali metal carboxylate, in the presence of a nitrogen-containing base as a catalyst, in an anhydrous medium and at a temperature of between 50 and 150 °C, with the formation of an allyl carboxylate, and then saponification of the latter with the aid of an alkali metal base, with the formation of allyl alcohol and an alkali metal carboxylate, characterised in that the water-immiscible solvent is a ketone forming an azeotrope with water, and in that then water is added to the reaction mixture in order to dissolve the alkali metal halide formed during the condensation, the aqueous solution of alkali metal halide is separated from the ketonic phase containing the allyl carboxylate, an aqueous solution of an alkali metal hydroxide is then added to the ketonic phase in order to saponify the allyl carboxylate by heating to a temperature of between about 50 °C and about 150 °C, the organic and aqueous phases of the saponification medium are separated, the allyl alcohol is separated off by distillation of the organic phase and the remaining ketone is optionally recycled, ketone is added to the aqueous phase of alkali metal carboxylate resulting from the separation and the water is removed by azeotropic distillation, and the resulting ketonic suspension of alkali metal carboxylate is finally recycled to the condensation stage.

2. Process according to Claim 1, characterised in that the alkali metal carboxylate is an alkali metal salt of an aliphatic acid having from 1 to 5 carbon atoms.

3. Process according to Claim 2, characterised in that the alkali metal carboxylate is sodium acetate or potassium acetate.

4. Process according to Claims 1 to 3, characterised in that the ketone is chosen from the group comprising diisobutyl ketone, pentan-3-one, methyl hexyl ketone, heptan-2-one, benzophenone and acetophenone.

## Ansprüche

1. Verfahren zur Herstellung von Allylalkoholen durch Kondensation, in Gegenwart eines organischen, mit Wasser nicht mischbaren Lösungsmittels, eines Allylhalogenids der allgemeinen Formel

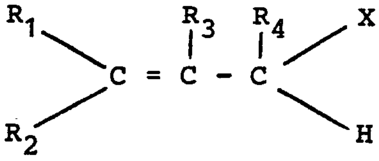

worin bedeuten

a) $R_1$, $R_2$, $R_3$, $R_4$, die identisch oder voneinander verschieden sind, bedeuten Wasserstoff, einen Alkyl- oder Alkenylrest,

b) einer der Reste $R_1$ oder $R_2$ hat 1 bis 20 Kohlenstoffatome, die anderen Reste $R_1$, $R_2$ und $R_3$, $R_4$ haben 1 bis 5 Kohlenstoffatome,

c) X ist ein Chlor, Brom- oder Jodatom, mit einem Alkalicarboxylat in Gegenwart einer Stickstoffbase als Katalysator in wasserfreiem Milieu und bei einer Temperatur zwischen 50 und 150 °C, unter Bildung eines Allylcarboxylats, dann Verseifung des letzteren mittels einer Alkalibase mit Bildung des Allylalkohols und eines Alkalicarboxylats, dadurch gekennzeichnet, daß das mit Wasser nicht mischbare Lösungsmittel ein Keton ist, das mit ihm ein Azeotrop bildet, daß man dann Wasser zu der Reaktionsmasse gibt, um das im Verlauf der Kondensation gebildete Alkalihalogenid aufzulösen, die wäßrige Alkalihalogenid-Lösung von der das Allylcarboxylat enthaltenden Ketonphase abtrennt, dann eine wäßrige Lösung eines Alkalihydroxids zu der Ketonphase zusetzt, um das Allylcarboxylat durch Erhitzen auf eine Temperatur zwischen etwa 50 und etwa 150 °C zu verseifen, die organischen und wäßrigen Phasen von dem Verseifungsmilieu trennt, den Allylalkohol durch Destillation von der organischen Phase trennt und das verbleibende Keton gegebenenfalls rezykliert, zu der wäßrigen Phase des Alkalicarboxylats, das sich von der Trennung ergibt, Keton zusetzt und das Wasser durch azeotrope Destillation entfernt und schließlich die so erhaltene ketonische Suspension des Alkalicarboxylats zu der Kondensationsphase zurückführt.

2. Verfahren nach Anspruch 1, dadurch gekennzeichnet, daß das Alkalicarboxylat ein Alkalisalz einer aliphatischen Säure mit 1 bis 5 Kohlenstoffatomen ist.

3. Verfahren gemäß Anspruch 2, dadurch gekennzeichnet, daß das Alkalicarboxylat Natriumacetat und Kaliumacetat ist.

4. Verfahren gemäß den Ansprüchen 1 bis 3, dadurch gekennzeichnet, daß das Keton ausgewählt ist aus der Gruppe gebildet von Diisobutylketon, Pentan-3-on, Methylhexylketon, Heptan-2-on, Benzophenon, Acetophenon.